# EUROPEAN PATENT APPLICATION

(11) **EP 1 236 801 A2**
(43) Date of publication of application: **04.09.2002**
(21) Application number: 02251259.4
(22) Date of filing: 25.02.2002
(51) Int. Cl.: C12N 15/82, A01H 5/00, A01H 5/10

(54) **Method of agrobacterium mediated plant transformation through treatment of germinating seeds**

(30) Priority: 26.02.2001 CN 01104185
(71) Applicant: The Agri-Biotechnology Research Center of Shanxi, Taiyuan, Shanxi Province 030031 (CN)
(72) Inventor: Sun, Yi, P.R. China 030031 (CN); Wang, Jingxue, P.R. China 030031 (CN); Liu, Shaoxiang, P.R. China 030031 (CN); Wang, Hui, P.R. China 030031 (CN); Li, Yi, P.R. China 100871 (CN)
(74) Representative: Davies, Gregory Mark

(57) **Abstract**

The invention relates to a plant transformation method through treating germinating plant seeds with an *Agrobaterium* strain carrying a foreign DNA fragment. In particular, germinating plant seeds were used as receptors, Ti (tumor inducing) plasmid harbored in an *Agrobacterium tumefaciens* strain carrying an inserted foreign DNA fragment was used as a gene donor. The germinating plant seeds were co-cultured with the *Agrobacterium* strain, during the process the foreign DNA fragment was transferred into the donor plant genome. PCR amplification and PCR-Southern hybridization verified that the foreign gene fragment has been transferred into the donor plant and can inherit to the next generation.

## Description

### Field of the invention

The present invention relates to a plant transformation method mediated by *Agrobacterium.* In particular, the invention relates to a plant transformation method, in which germinating plant seeds used as receptors were transformed with Ti (tumor inducing) plasmid harbored in an *Agrobacterium tumefaciens* strain and carrying an inserted foreign DNA fragment.

### Background of the invention

With the pressure of population increase, environmental pollution, and the area reduction of arable land and depletion of unrenewable resources, it is more and more urgent to improve crop yield and quality. In traditional breeding programs, crop yield improvements mainly rely on selecting elite genotypes in the progenies of artificial crossing and utilization of hybrid vigor. At the present stage, crop yield and quality improvements can no longer solely depend on artificial crossing and selecting afterwards. The rapid development of biotechnology based on the advances of molecular biology has become one of the major driving forces of developing agriculture, food, chemical material, pharmaceutical industries and environmental protection.

Although it has been only 18 years since the first transgenic plant was brought about in the 1983(EP0193259), the development of plant genetic engineering has already been very fruitful. Compared with the conventional cross breeding, the genetic engineering can enormously broaden the gene pool available to plant breeders. Scientists now can go beyond the borders of species, modifying plants directively, therefore, the plant breeding process could be greatly speeded up. Tremendous progresses have been made in the plant genetic engineering fields, producing many genetically modified crop (GMC) varieties (hybrids) with disease resistance, insect resistance, stress tolerance, improved quality and enhanced yield (EP0731170A1,US5349124). Many of them have already been commercialized.

A highly efficient transformation technique is a prerequisite for a successful plant genetic engineering program. The current transformation techniques can be categorized into three groups based on their principles: ① Direct genetic transformation using physical or chemical delivery systems, e.g., microprojectile bombardment, PEG, electroporation, liposome, or pollen tube pathway, etc.; ② Using the reproductive system or germ cells of plant per se, e.g. pollen, ovary, etc.; ③ *Agrobacterium* or virus mediated transformation techniques.
plant calli or protoplasts are used as receptors for most direct plant transformation methods, such as, PEG fusion, electroperation (US5384253) or microinjection, etc. Their most apparent advantages are that they do not have obvious host range, and not produce chimeric plants. But protoplast culture and plant regeneration from protoplasts are very complicated and tedious processes, and are still very difficult and inefficient for most plants.

Microprojectile bombardment is one of the most widely used DNA delivery systems. Gold or tungsten particles (approximately 1 um in diameter) are coated with DNA. The coated particles are accelerated to high speed (300-600 meters/second) with a special bombardment set. At these speeds, the particles can penetrate plant cell walls and membranes and introduce foreign DNA transfer. This technique can be used to introduce foreign DNA into plant cells. Once inside a cell, the DNA, by some unknown process, may integrate into plant genome. With the microprojectile bombardment system it is possible to transform a large number of different plant species, including monocots and conifers, plants that are considered not susceptible to *Agrobacterium*-mediated transformation. By this method one can introduce foreign DNA into plant cell suspensions, cullus cultures, meristematic tissues, immature embryos, coleoptiles, epicotyl and pollen in a wide range of different plants. But the bombardment apparatus and its supplies are relatively expensive, its transformation efficiency is relatively low, copy number of foreign DNA is uncontrollable, and high copy number of foreign DNA often causes silencing of the transferred foreign genes.

N. Xu et al (WO Patent 91/00358) reported a plant genetic transformation method with the assistance of sonication treatment (CN 1180746A).

Most above mentioned direct plant transformation methods use plant explants, e.g., cell suspension, calli, protoplasts, etc., as receptors and need to go through tedious in vitro tissue culture procedures, during which some mutations may be induced, and some transgenic plantlets may be lost in transplanting. Thus their transformation efficiencies are relatively low.

Germ cell transformation techniques use plant reproductive organ, i.e., pollen, ovary, egg, or fertilized zygotes, etc, as receptors, or use reproductive processes as avenues to deliver foreign genes, such as, pollen tube pathway, ovary injection, etc. Using plant reproductive system to deliver foreign DNA, one can circumvent tedious and often inefficient tissue culture, and get transgenic seeds directly. These methods are usually easy to apply, and are conducive to the combination of modern biotechnology and conventional plant breeding. Therefore, they are the very promising techniques. The major limitations of these techniques are that they can only be practiced around plant flowering time, and the mechanisms of DNA delivery and integration are remaining unclear for some of the methods.

The *Agrobacterium* mediation method is so far the most intensively studied and most widely used plant transformation method. The gram-negative soil bacterium *Agrobacterium tumefaciens* is a phytopathogen, which as a normal part of its life cycle, genetically transform plant cells. Its transformation power actually depends on the Ti (tumor inducing ) plasmid, which carries T-DNA (transferred DNA) sequence. Genetically modified Ti plasmids do not have their disadvantages of inducing crown gall tumors, but maintain their capacity of transforming plant cells.

The initial step in the infection process is the attachment of *A. tumefaciens* to a plant cell at the site of an open wound, where the bacteria respond to certain plant phenolic compounds such as acetosyringone and hydroxyacetosyringone, which are excreted by susceptible wounded plants. These small molecules (i.e., acetosyringone, hydrexyacetosyringone, etc) act to induce the activity of the virulence (*vir*) genes that are encoded on the Ti plasmid.

After Ti plasmid carrying cells of *A. tumefaciens* attach to a host plant cell and the *vir* genes are induced, the T-DNA is transferred into plant cells by a process that is thought to be similar to plasmid transfer from donor to recipient cells during bacterial conjugation. The gene (s) that are located within the T-DNA region are transferred to plant cells where they can be integrated into plant genome.

Compared with other plant transformation methods, the *Agrobacterium* mediation transformation method has the advantages of: ① the transformation efficiency is high; ② transferred genes are stably inherited; and ③ it can mediate relatively large piece of DNA transfer. Therefore, it is one of the most desirable transformation methods for many plants. Still, the use of this method is limited by the insusceptibility of many plants to the bacterium, for example most monocotyledonous plants are insusceptible to the bacterium. Since the major cereal crops are all monocots, it has special significance for promoting agriculture production to study transformation techniques that apply to monocots. Fortunately, protocols for transforming some monocotyledonous crops, such as rice, maize and wheat by *A. tumefaciens* have been devised by us. The addition of acetosyringone or analogues plays an important role in the protocols since it activates infection of *A*. *tumefaciens.*

In almost all *Agrobacterium* mediation protocols, plant tissue culture is a prerequisite though the explants vary from coleoptiles, leaf discs to young inflorescences and immature embryos. Tissue culture procedures are not only tedious and time-consuming, but also have some other advert effects, including inducing mutations, losing plants in transplanting, requiring some special equipments, etc.

### Description of the Invention

The object of the present invention is to provide a novel plant transformation method, i.e., using germinating seeds as the receptors of *Agrobacterium* transformation, which will circumvent the process of plant tissue culture and regeneration.

The devised technical scheme of the present invention involves that germinating plant seeds used as receptors are co-cultured with the donor, an *Agrobacterium* strain containing Ti plasmid with inserted foreign DNA sequence, so that the foreign DNA sequence is transferred into plant genome. The transformed seeds are then sown in fields or greenhouses in the same way as for normal seeds. Selection pressure can be applied to the seedlings of the treated seeds if a selection marker is included in the inserted foreign DNA sequence. Harvest seeds on the treated plants and detect transgenic plants by PCR, dot blot hybridization or Southern hybridization. The expression of the transferred gene is detected by Northern or Western hybridizations and field test.

In order to aid the *Agrobacterium* infection, the plant germinating seeds are wounded in and around the meristem part by a razor blade. The wounding should be moderate so that it will not harm the seeds too severely. Certain amount of acetosyringone is added to the bacterium suspension to further assist the infecting process.

Our experiments have shown that our proposed technical scheme is feasible and we successfully obtained maize transformants with the newly invented technique, implying that we have provided a new receptor system for the *Agrobacterium* mediated transformation.

The advantages of the present Invention are that germinating plant seeds were used as the receptors of *Agrobacterium* mediated genetic transformation, which circumvent the rigorous tissue culture conditions required by the conventional *Agrobacterium* mediated transformation, yet maintaining the other merits of the method. Seeds have natural capacity to establish normal plants, therefore, it is much easier to generate an entire plant from a seed than to regenerate a plantlet from a explant requested by the plant transformation methods requiring tissue culture. Meanwhile, the present invention does not need expensive equipments and relatively complicated plant tissue culture techniques. Further more, because seeds are easy to handle, experiments using them can be conducted whole year round without restriction of seasons, thus the time cycle for obtaining transgenic plants (seeds) will be reduced. Therefore, the present invention has provided a plant genetic transformation method which is simple, rapid, economical and easy to be adopted by conventional breeders who will integrate genetic engineering technology into their routine breeding programs.

### Description of the Figures

Figure 1. The physical map of the plasmid, pWM101S6. 35S: CaMV 35S promoter; RDV: RDV gene coding region; Hyg(R): hygromycine resistant gene; T-Border(R): the right border region of Ti plasimd; T-Border(L): the left border region of Ti plasmid.
Figure 2. A photograph of PCR analysis of total DNA from the transformed plants and CK. Lane 1: molecular marker; Lane 2: jinhuang 96B (CK); Lane 3: plasmid; Lanes 4-6: various transformed T₁ plants of jinhuang 96B.
Figure 3. A photograph of PCR-Southern blot hybridization of total DNA from transformed plants and CK. Lane 1: plasmid ; Lane 2: C649 (CK); Lanes 3-10: various transformed T₁ plants of C649.

### Example

We firstly obtained a successful transformation on maize by using the transformation techniques of the present invention.

Maize is an important cereal crop, whose genetic transformation has been studied by many researchers around the world. It has been transformed by employing various methods including *Agrobacterium* mediation, microprojectile bombardment, electroporation, etc. All these methods require plant calli or even protoplasts as receptors for foreign gene delivery. Somaclonal variations are often brought about in the process from calli to regenerated plantlets, most of them are adverse, such as, sterility. Still more, regenerated plantlets often die prematurely or die in transplanting from test tubes to fields (greenhouses). All of above-mentioned adverse effects hindered the further development of maize transformation. The present invention has provided a simple, efficient novel genetic transformation technique for maize.

The following is a specific example of applying the present invention in the maize genetic transformation. It will be understood that the example is used only to describe the invention in detail, but not to limit the scope of the appending claims.

### 1. Stock Plants and Explants

Maize (*Zea mays*) inbred lines, C649, jinhuang 96B, jinhuang 96C and 478 were used as receptors, which were kindly provided by Mr. Su Shuwen of Crop Genetics Institute, Shanxi Academy of Agricultural Sciences, P. R. China. Normal seeds were immersed in water for about 24 hours in 25°C. Then a wound was made in the emerging embryos of each seed by a razor blade or a scalpel. Caution was taken that the wound is moderate and across the apical meristem of the appearing bud. The wounded seeds were co-cultured with *Agrobacterium tumefaciens.*

### 2. Agrobaterium tumefaciens Strain, Plasmid, and Culture

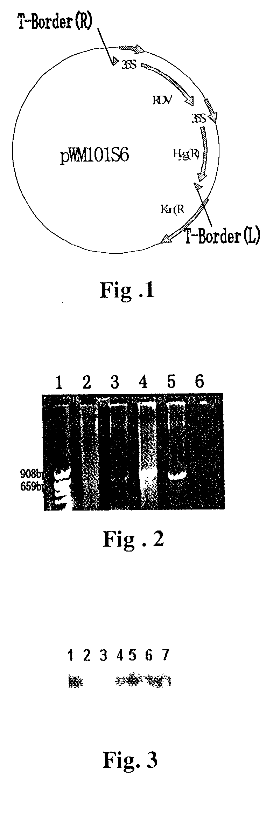

Disarmed *A. Tumefaciens **strain*** EHA101, harboring a binary vector pWM101S6 (Fig.1) was used for all the experiments. The vector contains a mutated RDV ( rough dwarf virus) moving protein gene and hygromycin resistance gene as a selection marker within T-DNA region. Each gene was under the control of a 35S promoter. The culture of *A. tumefaciens* was initiated from glycerol stock and grown overnight at 27 to 28°C with shaking (150rpm) in liquid Luria-Bertani medium(1% tryptone, 0.5% yeast extract, and 1% NaCI, pH7.0) containing 50 mg/L kanamycin to mid-log phase (OD₆₀₀=0.5-0.7). The *A. tumefaciens* cells were collected by centrifugation and resuspended in sterilized water with 100 µ mol/L acetosyringone. The *A. tumefaciens* cell density was adjusted to give an OD₆₀₀ of 0.04 to 0.06 for inoculation.

### 3. Inoculation and Co-culture

The wounded seeds were co-cultured with *A. tumefaciens* suspension and acetosyringone at 25 to 26°C with shaking (about 150 rpm) for about 48 hours.

### 4. Germination and Selection

After 48 hours, the seeds were cultivated on sterilized sand at 25 to 26°C and watered with the 25 µ g/L hygromycin solution. The seedlings with well-developed root systems and normal leaves were selected and transplanted into fields (greenhouses).

### 5. PCR amplification

According to the sequence of RDV gene , a pair of primers of 20bp was designed . The sequences of the primers were as following:
primer 1, 5'-AGGGTAAATCTCACAACATA-3';
primer 2, 5'-CGAAGCCAATCAATCACAGC-3'.

The primers were synthesized by Sangon Biotechnology Company , Shanghai , China . The fragment size between the two primers was 868 bp. The PCR reaction conditions were as following: 94°C, 4 min; 94°C, 0.75 min; 48°C, 0.75 min; 72°C, 1.75 min; 30 cycles; 72°C, 10 min . PCR amplification was made using TaKaRa TaqTM Kit and PTC-200 Thermal Cycler.

### 6. PCR-Southern blot hybridization

Following Wang and Fang (1998), a fragment in pWM101S6 was labeled with Dig-dUTP (PCR Dig Probe Synthesis Kit). PCR products of total DNA of transformed plants were fractioned with 1.2% agarose gel electrophoresis, and transferred to a nylon membrane, hybridized with Dig DNA Labeling and Detection Kit, detected with CSPD florescence stain and exposed to X-ray films.

### 7. Result

### 7.1 Seedling hygromycine resistance screening

In the spring of 2000, the total 3500 seeds were treated with the present inveted method, and 73 plants with hygromycine resistance were selected. The results were shown in Table 1.

**Table. 1**

| The seedling selection for hygromycine resistance | | |
|---|---|---|
| Inbred lines | Total numbers of treat seeds | No. of hygromycing resistance |
| Jinhuang 968 | 500 | 19 |
| Jinhuang 96C | 1000 | 29 |
| C649 | 1000 | 11 |
| 478 | 1000 | 14 |

The seedlings with hygromycine resistance were transplanted to fields 2 weeks after being screened. Most of the plants with hygromycine resistance grew normally. Two leaves of each plant were collected and stored at -40°C until DNA extraction. Maize ears were bagged before silking and selfed artificially. At the maturity total 45 selfed ears were harvested.

### 7.2 PCR amplification analysis

Total DNA of the 21 T₁ plants with hygromycin resistance was extracted and assayed by PCR amplification, of them 17 were shown positive and the results were shown in Fig. 2 and Table 2.

**Table 2.**

| The results of PCR amplification of T₁ plant | | |
|---|---|---|
| Inbred lines | No. Of assayed plants | No. of positive plants |
| C649 | 5 | 4 |
| Jinhuang 96B | 3 | 2 |
| Jinhuang 96C | 11 | 9 |
| 478 | 2 | 2 |
| Total | 21 | 17 |

### 7.3 PCR-Southern analysis

The T₁ seedlings of the above positive T1 plants were assayed again with PCR-Southern blot hybridization. All of them were positive (Fig .3). The results implied that the RDV gene was not only introduced into maize inbred lines, but also had been integrated into maize genome and passed on to the next generation. 7.4 Conclusion

The example demonstrated that the novel plant transformation approach we described here could be applied to maize successfully. In this example, the presence of the introduced RDV gene was detected in T₁ plants implying that the introduced gene could be integrated into the plant genome.

## Claims

1. A plant transformation method mediated by *Agrobacterium,* **characterized in that** germinating seeds were used as the receptors, an *Agrobacterium* strain containing Ti plasmids with an inserted nucleic acid sequence was used as the donor, the germinating seeds are co-cultured with the *Agrobacterium* strain so that the foreign nucleic acid sequence carried by the *Agrobacterium* strain can be transferred and integrated into the genome of the receptor through Ti plasmids.

2. The plant transformation method of claim 1, wherein said germinating seeds are wounded in and around their meristems of the appearing embryos.

3. The plant transformation method of claim 2, wherein said wounded germinating seeds are co-cultured with suspension of an *Agrobacterium* strain.

4. The plant transformation method of claim 3, wherein the medium inoculated by the *Agrobacterium* stain is added with the phenolic compounds selected from the group consisting of acetosyringone and hydroxyacetosyringone.

5. The plant transformation method of claim 3, wherein the medium inoculated by the *Agrobacterium* stain is added with the succus of dicotyledonous plants selected from the group of tobacco, tomato and potato.

6. The plant transformation method of any of claim 1 to 5, wherein said plant is a dicotyledonous plant.

7. The plant transformation method of any of claim 1 to 5, wherein said plant is a moncotyledonous plant.

8. The plant transformation method of claim 7, wherein said moncotyledonous plant is a maize.

9. The plant transformation method of claim 1, wherein said nucleic acid sequence is operably linked to a promoter.

10. The plant transformation method of claim 9, wherein said promoter is one selected from the group of a constitutive, inducible, viral, synthetic, and tissue-specific promoter.

11. A fertile transgenic plant produced by the method according to any of claims 1 to 10.

12. The transgenic plant of claim 11 which is a dicotyledonous plant.

13. The transgenic plant of claim 11 which is a moncotyledonous plant.

14. The transgenic plant of claim 13, wherein said moncotyledonous plant is a maize.

15. Seed from a transgenic plant according to any of claims 11 to 14.

16. A progeny plant from a seed obtained from a transgenic plant according to any of claims 11 to 14.

17. A germinating seed transformed according to the method of any of claims 1 to 10.
